# EUROPEAN PATENT SPECIFICATION

(11) **EP 1 848 441 B1**
(45) Date of publication and mention of the grant of the patent: **14.04.2010**
(21) Application number: 05777862.3
(22) Date of filing: 02.08.2005
(51) Int. Cl.: A61K 31/565, A61K 31/57, A61P 15/18

(54) **PHARMACEUTICAL COMPOSITION COMPRISING DROSPIRENONE AND ETHYNYLESTRADIOL**
PHARMAZEUTISCHE ZUSAMMENSETZUNG MIT DROSPIRENON UND ETHINYLÖSTRADIOL
COMPOSITION PHARMACEUTIQUE CONTENANT ÉTHINYLESTRADIOL ET DROSPIRÉNONE

(30) Priority: 09.08.2004 EP 04103837
(43) Date of publication of application: 31.10.2007
(73) Proprietor: Laboratorios Liconsa, S.A., 08028 Barcelona (ES)
(72) Inventor: SANDRONE, Carlos, Ariel, Capital Federal (AR); SAKSON, José, Mario, Capital Federal (AR); CAJARVILLE BASAISTEGUI, Maria del Carmen, Montevideo (UY); LARROSA POMI, José, Daniel, Canelones (UY)
(74) Representative: ZBM Patents
(86) International application number: PCT/EP2005/053759
(87) International publication number: WO 2006/015956

(56) References cited:
- EP-A- 1 216 712
- WO-A-01/15701
- US-A- 4 727 064

## Description

### FIELD OF THE INVENTION:

The present invention refers to a pharmaceutical composition that comprises drospirenone and ethynylestradiol with an improved dissolution rate. In addition, the invention refers to a method of preparation of a pharmaceutical formulation that comprises drospirenone and ethynylestradiol in order to improve its dissolution profile.
Said formulation can be used to produce an anovulatory effect when administered correctly in humans.

### BACKGROUND OF THE INVENTION:

It is known that the estrogenic and progestagen sexual hormones are practically insoluble in water or are so slowly dissolved that their biopharmaceutical properties are directly affected by this behavior or characteristic.

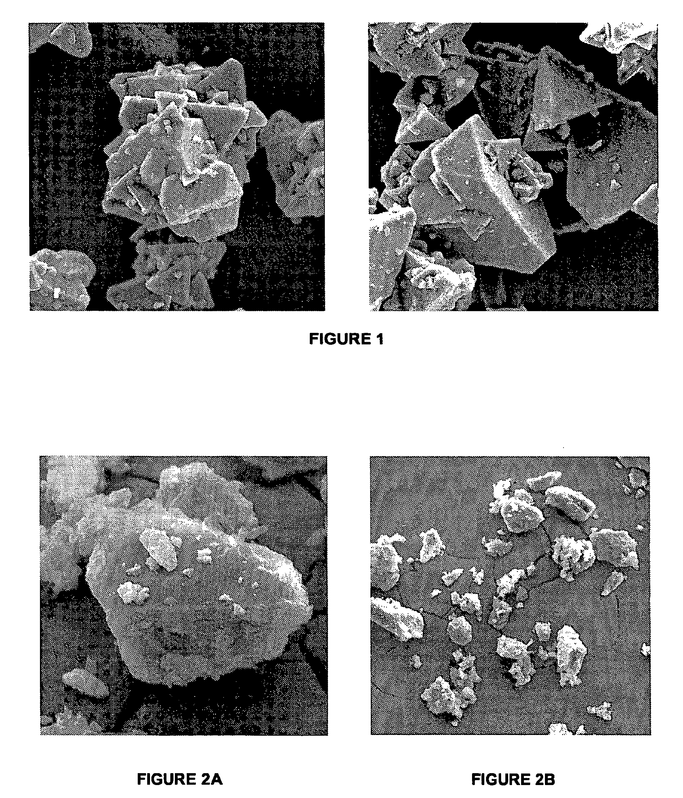

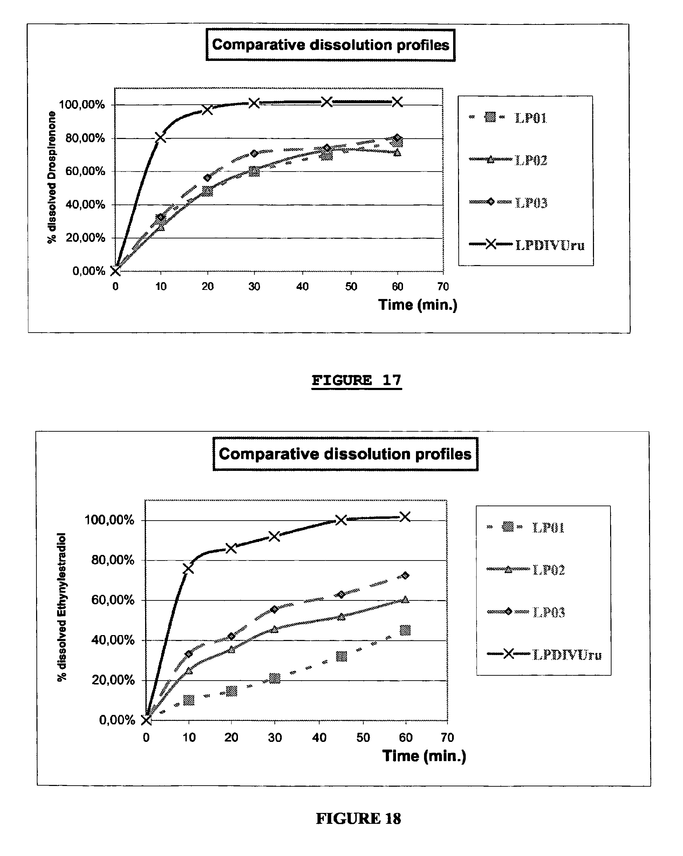

In particular, the synthetic progestagen drospirenone, or 6β,7β, 15β,16β-dimethylene-3-oxo-17α-pregn-4-ene-21,17-carbolactone, known from the patents DE3022337 and DE2652761, has a "spatula" form in its crystalline state as can be seen in the microphotographs in Figure 1 herein magnified 200x with a size no less than 50 microns. The dissolution rate of this crystal in oral pharmaceutical formulations such as tablets shows a very slow dissolution rate as shown in Figure 17 herein (see batches LP01, LP02 and LP03).

The published application EP1216712A1 discloses the preparation of inclusion complexes between cyclodextrin and drospirenone with the aim of increasing the solubility of drospirenone in water.

The bioavailability of a drug can be limited by a poor dissolution of the same in corporal aqueous fluids, particularly gastric fluids, immediately after administration. The dissolution rate can therefore be critical to rapidly reach therapeutically effective drug levels or in addition, as is the case for drospirenone, it can be influenced by the degradation of the active ingredient due to prolonged contact with gastric fluids with a very low pH, as described in the application WO01/15701 (in patent family with EP1214076B1).

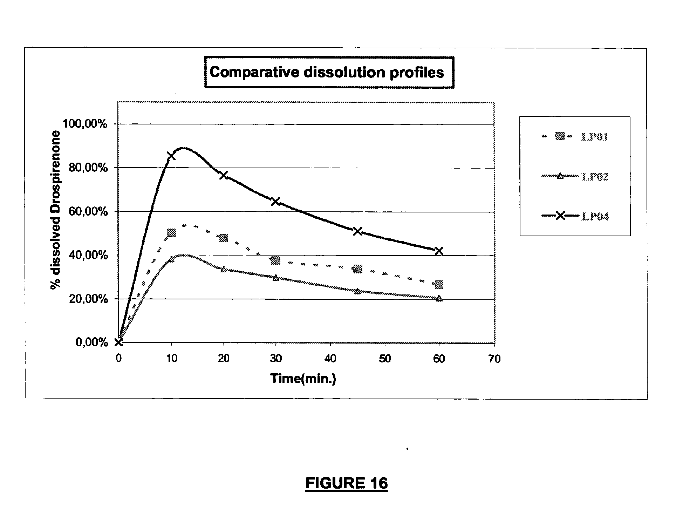

This behavior is seen *in vitro,* in an acidic medium (HCl 0.1 N), for samples of drospirenone with different particle sizes (see Figure 16 herein). It can be observed that the presence of the drug in the acidic medium decreases rapidly, after an initial dissolution, regardless of the size of the particles. Drospirenone's bioavailability is therefore doubly affected by the low dissolution rate in aqueous medium and by rapid degradation due to prolonged contact with gastric fluids with a very low pH.

The published patent EP1214076B1 discloses a pharmaceutical composition containing drospirenone and ethynylestradiol where the drospirenone is in a micronized form so that the particles of the active substance have a superficial area of more than 10.000 cm²/g, and a distribution of the size of the particles determined under a microscope where no more than two particles in a given batch have a diameter of more than 30 µm and preferably 20 particles or less have a diameter between 10 µm and 30 µm, thus increasing its dissolution rate particularly for use in oral contraception.

Further the final part of section [0017] of EP1214076B1 reads "Instead of providing the drospirenone in micronized form, it is possible to dissolve it in a suitable solvent, e.g. methanol or ethyl acetate, and spray it onto the surface of inert carrier particles followed by incorporation of the particles containing drospirenone on their surface in the composition." However, this section stands alone in the description of EP1214076B1. All working examples plus independent main claims refers to the micronized formulation.

### SUMMARY OF THE INVENTION:

The problem to be solved by the present invention is to provide a pharmaceutical composition of drospirenone and ethynylestradiol with an improved dissolution rate in water and bodily fluids and improved bioavailability.

The present inventors have discovered that with a pharmaceutical composition of drospirenone and ethynylestradiol in an amorphous form, without the presence of a crystalline state, they can improve the dissolution profiles of both active ingredients, thus increasing their bioavailability.

Further, the present inventors have demonstrated that use of the volatile solvent methylene chloride and in particular use of the combination of a mixture of the solvents methylene chloride and methanol gives SIGNIFICANT improved dissolution rate results over the use of methanol alone. For further details see e.g. Table 2 of Example 4 and Table 3 of Example 5 herein. Use of methanol alone is theoretically suggested in the prior art document EP1214076B1 (see above).

As explained herein, the volatile solvent methylene chloride is used to dissolve the drugs. In the subsequent granulation step there is dried until the residual levels of solvent is very low (see Example 1). However, even though it is low it is known to the skilled person that it is still possible to measure small amounts of the volatile solvent of interest in the final pharmaceutical composition. As known to the skilled person this may e.g. be done by use of gas chromatography (GC) and mass spectrometry (MS).

Therefore, the first aspect of the present invention refers to a pharmaceutical composition that comprises drospirenone and ethynylestradiol in an amorphous form, adsorbed on a therapeutically inert solid support, in combination with pharmaceutically acceptable excipients, characterized by that the composition comprises measurable amounts of the volatile solvent methylene chloride.

As said above, it is herein preferred to use a mixture of the solvents methylene chloride and methanol. Accordingly, in a preferred embodiment the composition of the first aspect is characterized by that the composition comprises measurable amounts of both of the volatile solvents methylene chloride and methanol.

The dissolution rate of drospirenone and ethynylestradiol is increased by introducing changes in their crystalline structure, so that the energy needed to dissolve the drug is much lower. In this case, the dissolution rate will increase considerably, thus achieving an increase in the drugs absorption rate. This way, the time the drugs are in contact with the gastric medium where the pH is too low is reduced, thus avoiding the eventual degradation reactions of the sensitive drugs by hydrolysis, as described above.

It is therefore an aim of the present invention to provide a pharmaceutical composition that contains drospirenone and ethynylestradiol in an amorphous form, without the presence of the crystalline state, preferably beginning with a non-micronized drug and using adequate dissolution/granulation solvents in its preparation.

Surprisingly, it has been discovered that the greatest dissolution rate is obtained by solubilization of drospirenone and ethynylestradiol in the appropriate combination of a mixture of the solvents methylene chloride and methanol and subsequent adsorption on adequate pharmaceutically inert excipients.
It has been observed that the use of another solvent, such as ethanol, even with heat, despite dissolving the drug, does not allow the application on pharmaceutically adequate excipients.
Further, use of methylene chloride and in particular use of the combination of a mixture of the solvents methylene chloride and methanol gives improved results over the use of methanol alone.

The new pharmaceutical composition of the invention is designed to provide a rapid dissolution of drospirenone and ethynylestradiol in water as the reference solvent.

More particularly, the invention provides a solid particulate composition that comprises drospirenone and ethynylestradiol in an amorphous state and adsorbed on a therapeutically inert support, with some components being soluble in water and others being insoluble in water, characteristics which facilitate a rapid disintegration of the pharmaceutical formulation.

Even more particular is an additional aim of the invention of a pharmaceutical composition that offers an improved dissolution of drospirenone and ethynylestradiol, which comprises a solid particulate formulation of an adsorbate consisting of drospirenone and ethynylestradiol in an amorphous state adsorbed on a solid, therapeutically inert, support, in combination with pharmaceutically acceptable excipients.

In a preferred embodiment, the solid particulate composition consists of particles selected from the following group of materials or adequate mixtures thereof: corn starch, pregelatinized starch, lactose, Croscarmellose sodic, yellow iron oxide and povidone covered with drospirenone and ethynylestradiol in an amorphous state.

Furthermore, another aim of the present invention is the preparation process of a pharmaceutical composition of drospirenone and ethynylestradiol, where said active compounds are found in an amorphous state and free of crystalline shapes.

Consequently, a second aspect of the present invention relates to a method for the preparation of a new pharmaceutical composition of drospirenone and ethynylestradiol, of the first aspect, that comprises following steps: i) dissolving drospirenone and ethynylestradiol in a volatile solvent or mixture of volatile solvents, ii) optionally adding a water-soluble polymer, iii) mixing until dissolution, iv) applying the resulting solution onto the solid, therapeutically inert support of particles, and v) drying the resulting adsorbate.
Preferably, at least one of the volatile solvent(s) of step (i) is methylene chloride. Preferably, there is added a water-soluble polymer in step (ii).

The active compounds drospirenone and ethynylestradiol, used in the preparation of the pharmaceutical composition of the invention are preferably in the form of non-micronized particles, more preferably with an average diameter no less than 50 µm and a specific surface area of less than approximately 5000 cm²/g measured by the BET technique.

Advantageously, the use of non-micronized forms reduces the environmental inconveniences associated with an additional stage in the manipulation of hormones, such as the process of micronization.

A third aspect of the invention relates to a pharmaceutical preparation that comprises a number of dose units for daily oral administration for a period of at least 21 consecutive days, where said dose units comprises approximately from 1 to 4 mg of drospirenone and approximately from 0,01 to 0,05 mg of ethynylestradiol, **characterized in that** said drospirenone and ethynylestradiol are in an amorphous form, adsorbed on a therapeutically inert solid support, of the first aspect of the invention or embodiments thereof as described herein, in combination with pharmaceutically acceptable excipients.

A fourth aspect of the invention relates to use of drospirenone combined with ethinylestradiol for preparing a pharmaceutical composition, of the first aspect of the invention or embodiments thereof as described herein, for the anovulation of a mammal, in particular a human, the composition comprising an amount of drospirenone corresponding to a daily dosage, on administration of the composition, of from about 1 mg to 4 mg, and comprising an amount of ethinylestradiol corresponding to a daily dosage, on administration of the composition, of from about 0.01 to 0.05 mg.

This fourth aspect may alternatively be formulated as a method for the anovulation of a mammal, in particular a human, that consists of administering a pharmaceutical composition, of the first aspect of the invention or embodiments thereof as described herein, made up of a number of dose units for daily oral administration, where said dose units consist of approximately 1 mg to 4 mg drospirenone and approximately 0,01 to 0,05 mg of ethynylestradiol, in which the drospirenone and ethynylestradiol are in an amorphous shape, adsorbed onto a solid therapeutically inert support, in combination with acceptable pharmaceutical excipients.

### BRIEF DESCRIPTION OF THE FIGURES:

Figure 1 shows the microphotographs of drospirenone in its crystalline state (magnified 200x)
Figure 2A shows a microphotograph of a lactose particle;
Figure 2B shows a microphotograph of a group of lactose particles;
Figure 2C shows a microphotograph of a particle of corn starch;
Figure 2D shows a microphotograph of a group of corn starch particles;
Figure 2E shows a microphotograph of a particle of pregelatinized starch;
Figure 2F shows a microphotograph of a group of pregelatinized starch particles;
Figure 2G shows a microphotograph of a particle of sodic Croscarmellose;
Figure 2H shows a microphotograph of a group of sodic Croscarmellose particles;
Figure 2I shows a microphotograph of a particle of yellow iron oxide;
Figure 2J shows a microphotograph of a group of yellow iron oxide particles;
Figure 2K shows a microphotograph of a particle of Povidone;
Figure 2L shows a microphotograph of a group of Povidone particles;
Figure 3 shows a microphotograph of a physical mixture of the two distinct components of the adsorbate;
Figures 4A and 4B show microphotographs of the adsorbate of drospirenone and ethynylestradiol on the components that are adequate for obtaining the tablets.
Figure 5 shows a graph of the DSC of lactose alone;
Figure 6 shows a graph of the DSC of drospirenone;
Figure 7 shows a graph of the DSC of magnesium stearate;
Figure 8 shows a graph of the DSC of corn starch;
Figure 9 shows a graph of the DSC of pregelatinized starch;
Figure 10 shows a graph of the DSC of yellow iron oxide;
Figure 11 shows a graph of the DSC of sodic Croscarmellose (Ac-Di-SoD)
Figure 12 shows a graph of the DSC of Povidone;
Figure 13 shows a graph of the DSC of placebo (mixture of the excipient components with the absence of ethynylestradiol and drospirenone)
Figure 14 shows a graph of the DSC of a simple mixture of the components of the adsorbate.
Figure 15 shows a graph of the DSC of the granules of Example 1 which contains drospirenone and ethynylestradiol adsorbed on the inert excipients.
Figure 16 shows the dissolution profiles for the tablets of drospirenone in a strongly acidic aqueous medium HCl 01 N.
Figure 17 shows the dissolution profiles for the tablets of the invention containing amorphous drospirenone adsorbed on inert excipients, compared to those of compositions containing crystalline drospirenone of various-sized particles.
Figure 18 shows the dissolution profiles for the tablets of the invention containing amorphous ethynylestradiol adsorbed on inert excipients, compared to those of compositions containing crystalline ethynylestradiol of various-sized particles.

### DETAILED DESCRIPTION OF THE INVENTION:

### A pharmaceutical composition comprising drospirenone and ethynylestradiol and a method for preparing it:

In agreement with the first aspect, the present invention provides a solid particulate formulation that consists of drospirenone and ethynylestradiol in an amorphous state adsorbed on a pharmaceutically acceptable and therapeutically inert support.

The term "adsorbed on a therapeutically inert solid support" may herein also be termed "adsorbed on a therapeutically inert solid base" and the terms may herein be used interchangeably.

Preferably, the solid particulate formulation contains particles of pharmaceutically acceptable excipients covered with drospirenone and ethynylestradiol in an amorphous state and ready to be compressed into their definitive pharmaceutical formulation.

The composition of the invention can be obtained according to the following steps:
i) dissolving drospirenone and ethynylestradiol in a mixture of volatile solvents, ii) adding a polymer that is water-soluble and/or soluble in the volatile solvent or mixture of volatile solvents, iii) mixing until dissolution, iv) applying the resulting solution onto a support of pharmaceutically acceptable and therapeutically inert solid particles, v) drying the resulting adsorbate.

The volatile solvent may be methanol. However, preferably, the volatile solvent is methylene chloride and even more preferable is a mixture of methylene chloride and methanol, which is acceptable to use in drug manufacturing processes.

Preferably the volatile solvent is a [9 to 4]:[3 to 0.5] v/v mixture of methylene chloride/methanol. Within the range of mixtures it is preferred that the volatile solvent is a 6:1 v/v mixture of methylene chloride/methanol or a 5:2 v/v mixture of methylene chloride/methanol.

The drospirenone can be prepared as described, in for example, US 4.129.564. The drug drospirenone used in the preparation of the pharmaceutical composition of the invention is preferably in the form of non-micronized particles with an average diameter of no less than 50 µm and a specific surface area of less than approximately 5000 cm²/g obtained by the BET technique.

The estrogen ethynylestradiol can be prepared according to the methods described in GB 516.444, US 2.243.887, US 2.251.939, US 2.265.976 and US 2.267.257. The drug ethynylestradiol used in the preparation of the pharmaceutical composition of the invention is preferably in the form of non-micronized particles with an average diameter no less than 50 µm and a specific superficial area obtained by the BET Technique of less than approximately 5000 cm²/g.

Water-soluble polymers that are acceptable for the preparation of the binder solution are preferably chosen from the polyvinylpyrrolidones as preferred in the method of the invention, more preferably polyvinylpyrrolidone Povidone K 30 (such as that provided by BASF or ISP).

Examples of other suitable water-soluble polymers include a polymer selected from the group consisting of: Alginic acid, Carbomer, Carboxymethylcellulose Sodium, Carrageenan, Dextrin, Hydroxypropyl Methylcelullose, Polyethylene Glycol, Polymethacrylates, Polyvinil Alcohol and Povidone K90.

The resulting solution is applied onto a base of solid particles, which are pharmaceutically acceptable and therapeutically inert. The application is preferably done using a mixer/granulator like a Pony mixer. However, it may also be done by other techniques such as fluid bed based spraying.

The adequate particle materials used to make the base of the invention are preferably chosen from either starch, pregelatinized starch, lactose, sodic Croscarmellose, or yellow iron oxide and mixtures of the same.

The lactose used is preferably monohydrate lactose, which is commonly used as an excipient and diluent in the pharmaceutical industry. This lactose can be provided by manufacturers such as MEGGLE, QUEST International, or BORCULO Whey Products.

The starch can be from corn or potatoes, and is commonly used as a diluent in the pharmaceutical industry. The starch can be provided by: ORCON, OAVEBE, CERESTAR INT. SALES, GRAIN PROCESSING CORP., PARTICLE DYNAMICs INC.,GLUTAL, etc.

The pregelatinized starch can be from corn or potatoes, and is commonly used as a diluent and agglutinant in the pharmaceutical industry. The pregelatinized starch can be provided by: COLORCON, OAVEBE, CERESTAR INT. SALES, GRAIN PROCESSING CORP., PARTICLE DYNAMICs INC.,GLUTAL, etc.

The sodic croscarmellose, commonly used as a superdisintegrants in the pharmaceutical industry, can be provided by COLORCON, ALLCHEM INTERNATIONAL, METSA SPECIALITY CHEMICAL LTD, AVEBE, etc.

The yellow iron oxide, commonly used as a coloring material in the pharmaceutical industry, can be provided by COLORCON.

The magnesium stearate, commonly used as a lubricant in the pharmaceutical industry, can be provided by LE STAR QUIMICA.

The granules obtained can be dried using any known drying method within the art, preferably the drying may be done in a static bed dryer.

The drying is carried out until obtaining a granule with residual levels of solvent that are 100 times below that permitted by the International Harmonization Conference on technical requirements for the registration of pharmaceutical products for human use, in its guide Q3C.

By subjecting the granule to drying, the presence of polyvinylpyrrolidone in the drospirenone and ethynylestradiol solution acts by the formation of a tridimensional polymer structure, preventing the crystalline growth of drospirenone and ethynylestradiol, in such a way that the drugs are deposited onto the excipient particles in an amorphous state. Subsequently, this mixture should only be lubricated in order to compress it and obtain a pharmaceutical formulation that gives the dose unit.

A separate aspect of the invention relates to a pharmaceutical composition that comprises drospirenone and ethynylestradiol in amorphous form, adsorbed on a therapeutically inert solid support, in combination with pharmaceutically acceptable excipients obtained by a method for the preparation of a pharmaceutical composition of the second aspect of the invention and embodiments thereof as described herein.

Particular examples of preparation are given below. The purposes of these are merely illustrative and are not intended to limit the scope of the invention, which is determined by the claims.

### Pharmaceutical preparation:

Preferably, the pharmaceutical preparation of the third aspect of the invention is characterized because it comprises of drospirenone and ethynylestradiol in amorphous form, adsorbed on a solid basis chosen from among particles selected from the group consisting of corn starch, pregelatinized starch, lactose, sodic croscarmellose, yellow iron oxide, polyvinylpyrrolidone or mixtures of one or more of the same, in combination with pharmaceutically acceptable excipients.

Further, the pharmaceutical preparation is preferably characterized because at least 80% of drospirenone dissolves from said dose units within 20 minutes, in a test carried out in a dissolution apparatus according to the U.S. Pharmacopoeia, Edition number 27, Apparatus 2, in 900 ml of distilled water at 37°C and stirred at 50 rpm.

In a further embodiment the pharmaceutical preparation is preferably characterized because at least 80% of ethynylestradiol dissolves from said dose units within 20 minutes, in a test carried out in a dissolution apparatus according to the U.S. Pharmacopoeia, Edition number 27, Apparatus 2, in 900 ml of distilled water at 37°C and stirred at 50 rpm.

Compositions particularly adequate for oral administration are unitary dose formulations such as tablets, capsules, pills, powder in packets, etc. These dose units will normally contain an amount of drospirenone in the range from approximately 1 mg to approximately 4 mg, with 3 mg being preferable, and an amount of ethynylestradiol in the range from approximately 0,01 to approximately 0,05 mg, with 0,02 to 0,03 mg being preferable.

### A method for the anovulation a mammal:

This fourth aspect may alternatively be formulated as a method for the anovulation of a mammal, in particular a human, that consists of administering a pharmaceutical composition, of the first aspect of the invention or embodiments thereof as described herein, made up of a number of dose units for daily oral administration, where said dose units consist of approximately 1 mg to 4 mg drospirenone and approximately 0,01 to 0,05 mg of ethynylestradiol, in which the drospirenone and ethynylestradiol are in an amorphous shape, adsorbed onto a solid therapeutically inert support, in combination with acceptable pharmaceutical excipients.

With respect to the method for the anovulation of a mammal, in particular a human, of the fourth aspect of the invention, the daily oral administration is preferably for a period of at least 21 consecutive days.

Further, there may be 7 or less said daily dosage units containing no active agent.

Alternatively, it is possible to include, in the dosage regimen, a period of 7 days or less during which no dosage units are ingested.

In suitable embodiments, the number of daily dosage units comprising the combination of drospirenone and ethinylestradiol may be 21, 22, 23 or 24, and the number of daily dosage units containing no active agent may then be 7, 6, 5 or 4, as the case may be.

Alternatively, the a pharmaceutical composition, of the first aspect of the invention or embodiments thereof as described herein, may be used for hormone replacement therapy (HRT). The skilled person knows how to optimise doses and administration with respect to hormone replacement therapy.

### Pharmaceutically acceptable excipient

By "pharmaceutically acceptable excipient" it should be understood as any pharmaceutically acceptable conventional vehicle including binders, tablet desintegrants, diluents, lubricants, tablet glidants, opacifiers of tablets and capsules, colorings, sweeteners, flavoring, antioxidants, buffer agents, etc.

As used here, the expression "binders" designates substances used to provoke the adhesion of the dust particles in the granules of the tablets, and include as examples and without being limiting, sodic carboxymethylcellulose, polyvinylpyrrolidone, sugars, ethylcellulose, gelatin, guar gum, polyethylene glycol, polyethylene oxide or combinations of the same and similar ones known to those with common knowledge of the art.

As used here, the expression "tablet desintegrants" designates a compound used in solid dosing formulations to promote the rupture of the solid mass into small particles. Examples of desintegrants include, without being limiting, starches, pregelatinized starches, bentonite, microcrystalline cellulose and its esters and salts, calcium or sodium carboxymethylcellulose and its modifications, alginates, reticulated povidone, gums such as guar, agar, pectin and the like that are known to those with common knowledge of the art.

As used here, the expression "tablet diluents or filler" designates inert substances used as filling materials to create the desired mass, in the preparation of tablets and capsules, and include, as examples and without being limiting, dibasic calcium phosphate, kaolin, lactose, saccharose, mannitol, microcrystalline cellulose, sorbitol, starch and sugars (mono and polysaccharides) and the like known to those with common knowledge in the art.
As used here, the expression "lubricant" designates substances used in the formulations of tablets to reduce friction during the compression of the tablets, that include, as examples and without being limiting, calcium stearate, magnesium stearate, mineral oil, stearic acid, 1-Leucine, zinc stearate, talc and the like that are known by those with common knowledge in the art.

As used here, the expression "tablet glidants" or "flow promoters" designates agents used in the formulations of capsules and tablets to promote the fluidity of the granulation, that include, as examples and without being limiting, colloidal silica, corn starch, talc calcium silicate, magnesium silicate, and the like that are known by those with common knowledge in the art.
As is used here, the expression "opacifiers of tablets and capsules" designates a compound used to coat a tablet or capsule with an opaque covering combined or not with a coloring, and include, as examples and without being limiting, titanium dioxide, magnesium carbonate, calcium carbonate and the like that are known by those with common knowledge of the art.

As is used here, the expression "coloring" designates a compound used to give color to the pharmaceutical compositions, as for example and without being limiting, caramel, ferric oxide, beta carotene, carmine, curcumin, coloring bases and their sprays and other materials known by those with common knowledge in the art.

As used here, the expression "sweetening agent" designates a compound used to give a preparation a sweet flavor, and include as examples without being limiting, saccharose, dextrose, aspartame, mannitol, sodium saccharin, sorbitol and the like known by those with common knowledge of the art.

As is used here, the expression "flavoring" designates a compound used to give flavor and/or a pleasant aroma to a pharmaceutical preparation. Examples of flavoring agents include, without being limiting, flavoring and aromatic oils, synthetic flavorings and/or natural oils, and plant extracts, leaves, flowers, fruits and other combinations of the same.

As is used here, the expression "antioxidant" designates an agent that prevents the oxidation and therefore the deterioration of the active agent and other components by oxidative processes, and include as examples without being limiting ascorbic acid, ascorbyl palmitate, sodium bisulfate and the like known by those with common knowledge of the art.

As used here, the expression "buffer agent" designates a compound used to resist the change in pH when there is dissolving or the addition of acids or alkalines in the composition, and include as examples without being limiting, potassium metaphosphate, potassium phosphate, monobasic sodium acetate, sodium citrate and the like known by those with common knowledge of the art.

It should be understood that the compounds used in the art of pharmaceutical formulations generally serve a variety of functions and objectives, meaning that the agents mentioned are therefore not for a specific function or limited to that function.

### Separate independent aspects of the invention

As explained herein and demonstrated in working examples herein (see e.g. Table 2 of Example 4 and Table 3 of Example 5) a pharmaceutical composition as described herein may be characterized by a very good dissolution profile. More specifically, it may be characterized by that at least 80% of drospirenone and ethynylestradiol dissolves from said composition within 20 minutes in a test carried out in a recognized standard dissolution apparatus.

This is the first time that a pharmaceutical composition has been described with such a preferred dissolution profile and it is a significant contribution to the art. As explained herein, such a pharmaceutical composition is preferably obtained by use of methylene chloride as one of the volatile solvents. However once the skilled person now knows that one may be able to obtain such a pharmaceutical composition he could further investigate the issue and probably identify other solvents than methylene chloride that could be used to make adequate pharmaceutical compositions.

Accordingly, a separate independent aspect of the invention relates to a pharmaceutical composition that comprises drospirenone and ethynylestradiol in amorphous form, adsorbed on a therapeutically inert solid support, in combination with pharmaceutically acceptable excipients, **characterized in that** at least 80% of drospirenone and at least 80% of the ethynylestradiol dissolve from said composition within 20 minutes in a test carried out in a dissolution apparatus according to the U.S. Pharmacopoeia, Edition number 27, Apparatus 2, in 900 ml of distilled water at 37°C and stirred at 50 rpm.

As discussed above, the pharmaceutical composition of this separate independent aspect of the invention may be made by use of methylene chloride as volatile solvent. However, it may also be made by use of other volatile solvent(s) not involving methylene chloride.

In line of this, another separate independent aspect of the invention relates to a method for the preparation of a pharmaceutical composition, of above mentioned separate independent aspect of the invention that comprises following steps:
i) dissolving drospirenone and ethynylestradiol in a volatile solvent or mixture of volatile solvents,
ii) optionally, adding a water-soluble polymer,
iii) mixing until dissolution,
iv) applying the resulting solution onto a base of solid particles that are therapeutically inert,
v) drying the obtained granulated adsorbate.

Preferably, there is in step ii) added a water-soluble polymer.

The working examples herein provide guidance in order to identify other (not methylene chloride) adequate volatile solvents. For instance, in comparative example 2B is shown that 16 ml of methanol alone is not enough to completely dissolve 3.06g of drospirenone. This may be used as basis for identifying suitable solvents. One may repeat the dissolution protocol, as described in working example 1 herein, but using a panel of other suitable solvent candidates. The ones that are capable of completely dissolving drospirenone are herein preferred solvents.

Accordingly, a preferred embodiments of the method of the separate independent aspect above, is wherein the volatile solvent or mixture of volatile solvents of step i) is/are solvent(s) that are capable of completely dissolving drospirenone in the following dissolution assay:
16.0 ml of the volatile solvent or mixture of volatile solvents are placed in a stainless steel stirring apparatus, next, 0.0306 g of ethynylestradiol (non-micronized with a particle size no less than 50 µm) are added under constant stirring, the mixture is shaken/stirred until completely dissolved, next 3.06 g of drospirenone (non-micronized with a particle size no less than 50 µm) are added under constant stirring until the drospirenone is completely dissolved.

All embodiments described above in relation to the aspects of the invention described above are also preferred embodiments in relation to the separate independent aspects of the invention as described in this section.

### EXAMPLES:

### Examples for Reference

### Dissolution profiles of crystalline drospirenone in acidic medium

The dissolution profiles were obtained from tablets containing drospirenone in a crystalline state with different sized particles, using a dissolution apparatus designated in the U.S. Pharmacopoeia, Edition 27, apparatus 2, at 50 rpm and 900 ml of lauryl sulfate 0,07% in a strongly acidic medium, HCl 0,1 N, thermostatized at 37°C. The results of the amounts of dissolved drug at different time-points are summarized in the following table:

**Table 1**

| | % of drospireno ne dissolved | | | | | |
|---|---|---|---|---|---|---|
| Time (minutes) | 0 | 10 | 20 | 30 | 45 | 60 |
| LP01 | 0 | 50,1% | 47,9% | 37,6% | 33,8% | 26,7% |
| LP02 | 0 | 38,1% | 33,5% | 29,7% | 23,8% | 20,5% |
| LP04 | 0 | 85,3% | 76,5% | 64,6% | 51,1% | 42,2% |

### Samples:

LP01: Batch of tablets with non-micronized, crystalline drospirenone with an average particle size of> 100 µm
LP02: Batch of tablets with non-micronized, crystalline drospirenone with an average particle size = 100 µm
LP04: Batch of tablets with micronized, crystalline drospirenone with an average particle size = 10 µm

For a better interpretation of the results shown in Table 1, the results are also shown in Figure 16. From this figure one can see the instability of the drospirenone in an acidic medium, regardless of the size of the particle.

### EXAMPLE 1

Amount required for producing 1000 tablets:

### Granule:

| | |
|---|---|
| Drospirenone (+ 2% excess for losses during the process) | 3,0600 g |
| Ethynylestradiol(+ 2% excess for losses during the process) | 0,0306 g |
| Corn starch | 12,7800 g |
| Pregelatinized starch | 15,4400 g |
| Monohydrate lactose | 44,0000 g |
| Sodic croscarmellose | 0,4000 g |
| Yellow iron oxide | 0,0900 g |
| Povidone | 3,4000 g |
| Methylene chloride / methanol (6:1) | 16,0000 ml |
| | |
| *Lubrication and final mixture:* | |
| Sodic Croscarmellose | 0,4000 g |
| Magnesium stearate | 0,4000 g |
| *Covering*/*coating:* | |
| Aqueous clear lake 5% | 0,0060 g |
| (OPADRY YS-1-7006 at 5%) | |

### II. PREPARATION TECHNIQUE

### II.1. Dissolution of the active ingredients and binder

16,00 ml of the dichloromethane/methanol solution at a 6:1 ratio were placed in a stainless steel stirring apparatus . Next, 0.0306 g of ethynylestradiol, non-micronized with a particle size no less than 50 µm (addition of an excess of 2% was foreseen to make up for losses during the process), were added under constant stirring. The mixture was shaken/stirred until completely dissolved. 3.0600 g of drospirenone, non-micronized with a particle size no less than 50 µm (addition of an excess of 2% was foreseen to make up for losses during the process), were added under constant stirring until completely dissolved. Once dissolved, 3.4000 g of polyvinylpyrrolidone K-30 were added with stirring until completely dissolved.

### II.2. Granulation - Drying

In a planetary-type mixer, the following, which were previously adequately sized by a 30 mesh net, were added: 44.0000 g of monohydrate lactose, 12.7800 g of corn starch, 15.4400 g of pregelatinized starch, 0.4000 g of sodic croscarmellose and 0.0900 g of yellow iron oxide. These were mixed for 15 to 20 minutes.

Next, the previously prepared binder solution that contains the active ingredients was added, and they were kneaded until reaching an adequate consistency for granulating. If necessary, it is possible to add additional solvent (mixture 6:1 of dichloromethane/methanol).

The wet mass was granulated by making it pass through a 10 mesh net. It was dried in a heater at 40°C for 24 hours, until reaching residual levels of solvent that were 100 times less than that allowed by the International Conference of Harmonization of technical requirements for the registration of pharmaceutical products for human use, its guide Q3C.

In a separate experiment, the binder solution was applied onto the base of solid particles via spraying. It was done in the following way. In a fluid bed dryer / granulator, previously sized by a 30 mesh, were added: 44.0 g of monohydrate lactose, 12.780 g of corn starch, 15.44 g of pregelatinized starch, 0.400 g of sodic croscarmellose and 0.0900 g of yellow iron oxide. These were mixed for 15 to 20 minutes. Then, the previously prepared binder solution that contains the active ingredients was sprayed. The wet mass was dried at 40°C.

### II.3. Final Mixture - Compression - Covering

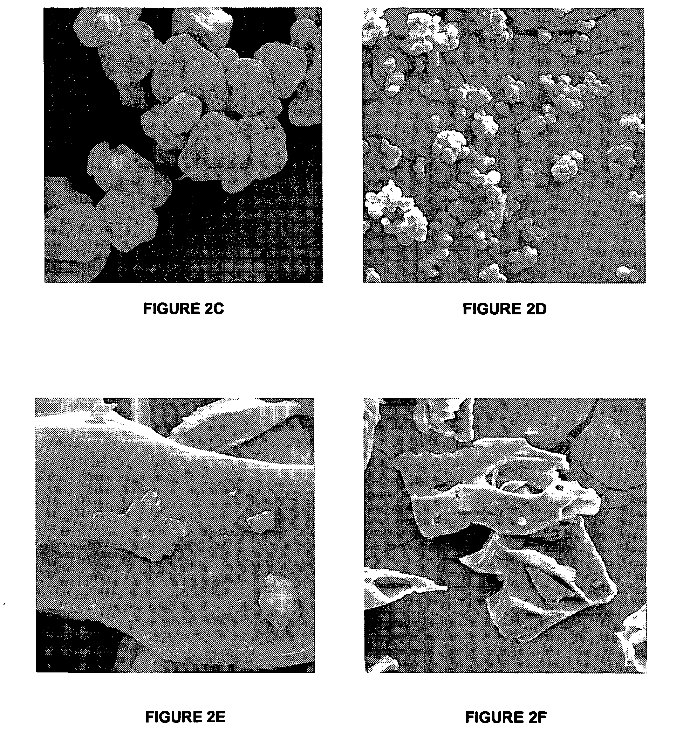

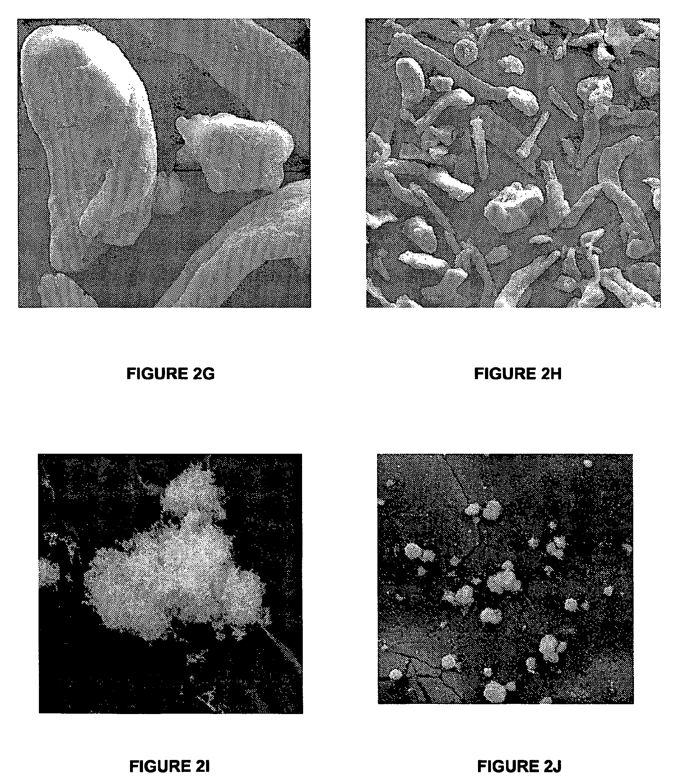

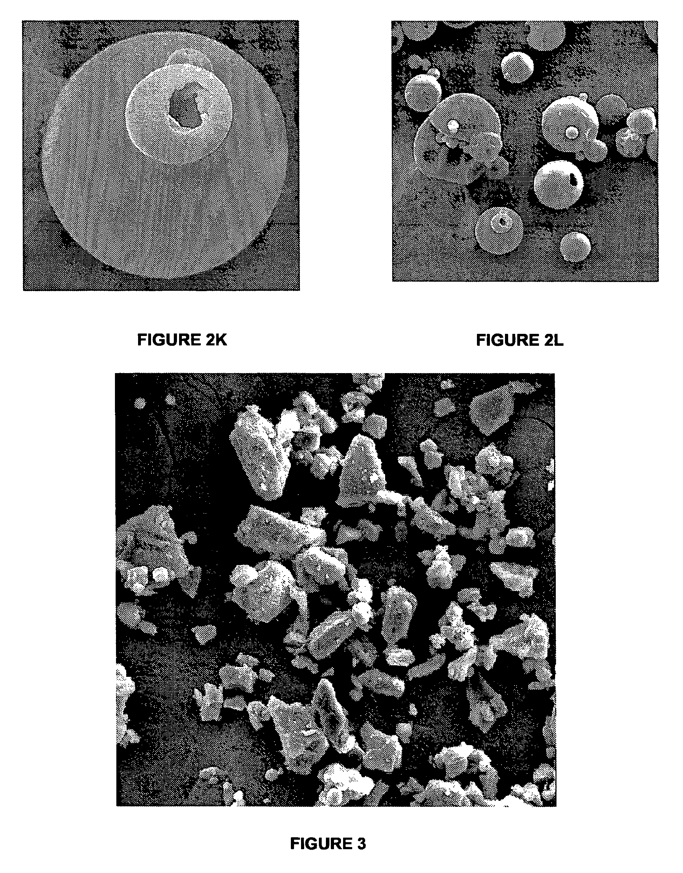

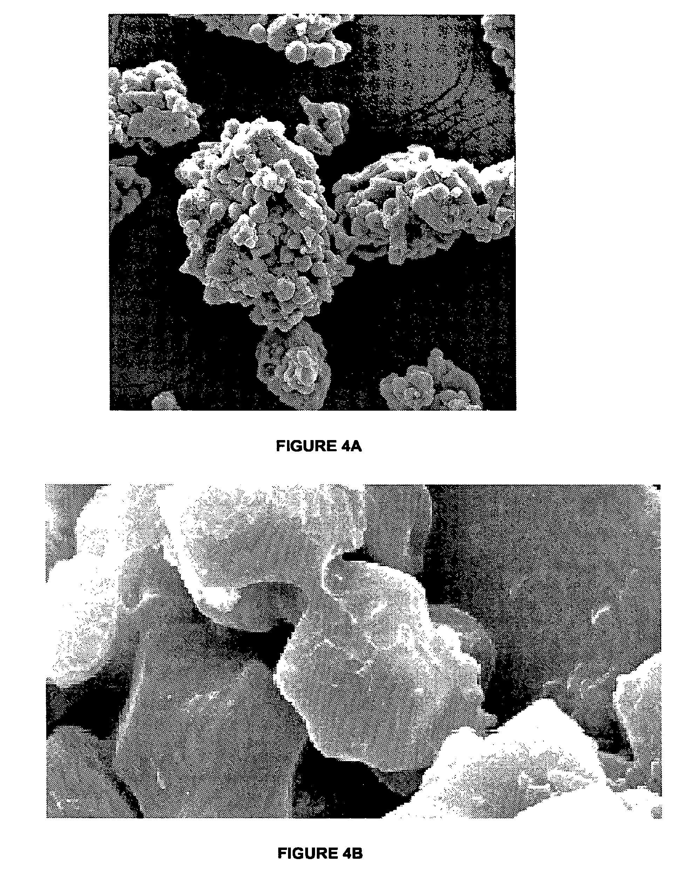

The dried granulate was passed through net 18, followed by the addition of 0,4000 g of sodic croscarmellose and 0.4000 g of magnesium stearate. These were mixed for 15 minutes. The resulting granules were compressed into tablets using a rotating machine equipped with a set of flat biconcave punchers of 13/64" in diameter at a weight of 80 mg/tablet and a force of 6-12 Stron Cobb units.
The compressed tablets were coated with an aqueous clear lake 5%. Name of the batch granulated in the planetary-type mixer: LPDIVuru and name of the batch granulated in the fluid bed dryer: LPDISpray.
The morphology of the components in each step of the process has been analyzed under an electronic microscope. The microphotographs in Figure 1, magnified 200x, show crystals of drospirenone, with its characteristic "spatula" shape at sizes greater than 50 µm. Figures 2A to 2L show microphotographs of the therapeutically inert materials used as a base for the active agents. In particular, Figures 2A and 2B show microphotographs of a lactose particle and of a group of lactose particles, respectively; Figures 2C and 2D show microphotographs of a corn starch particle and a group of corn starch particles, respectively; Figures 2E and 2F show microphotographs of a particle of pregelatinized starch and a group of pregelatinized starch particles, respectively; Figures 2G and 2H show microphotographs of a particle of sodic croscarmellose and a group of sodic croscarmellose particles, respectively; Figures 2I and 2J show microphotographs of a particle of yellow iron oxide and a group of yellow iron oxide particles, respectively; Figures 2K and 2L show microphotographs of a particle of Povidone and of a group of Povidone particles, respectively. Figure 3 is a microphotograph of a physical mixture of the different components. Figures 4A and 4B are microphotographs of particles of the adsorbate of drospirenone and ethynylestradiol on the inert components, in two views.

### EXAMPLE 2

A batch of 1000 tablets were prepared using a process similar to that described in Example 1, where the mixture of volatile solvents methylene chloride / methanol were applied at a proportion of 5:2. Results similar to those described in Example 1 were obtained.

### EXAMPLE 2B

This is a comparative example, where only methanol was used as solvent. Only the differences compared to example 1 is given below. The rest was done identical to example 1.

In the first example was used 16 ml methanol only. The 16 ml is the same volume as the 16 ml methylene chloride / methanol (6:1) used in example 1. When made the step "*II.1. Dissolution of the active ingredients and binder*", it is important to note that this 16 ml quantity of methanol was not enough for the total dissolution of drospirenone because the methanol solubility was to low. Recall that in example 1 drospirenone was complete dissolved. However, even though the process was continued in accordance with example 1, where there in step "*II.2. Granulation - Drying*" was used a planetary-type mixer and kneaded. The name the final batch granulated: LPDIMet-16ml.

In view of above was made an extra experiment, where there in step step "*II.1. Dissolution of the active ingredients and binder*" was added methanol until complete dissolution. The required amount was 154.3 ml methanol. Such a high necessary amount of methanol is generally excessive for a relevant industrial granulation process. The process was continued in accordance with example 1, where there in step "*II.2. Granulation - Drying*" was used a fluid bed dryer and sprayed. The name the final batch granulated: LPDIMet-154ml.

### EXAMPLE 3

### Differential Scanning Calorimetry (DSC)

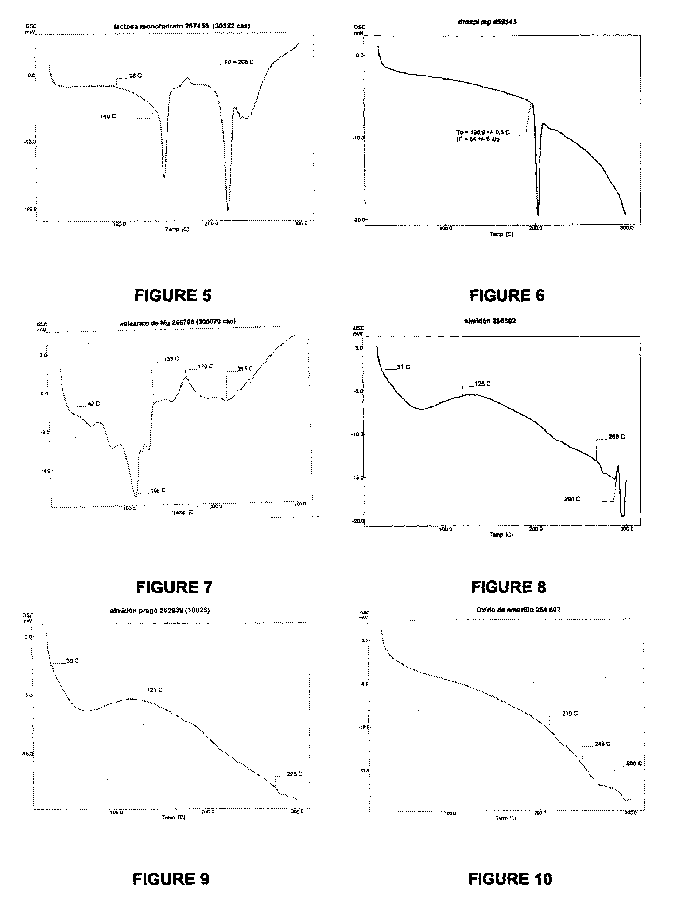

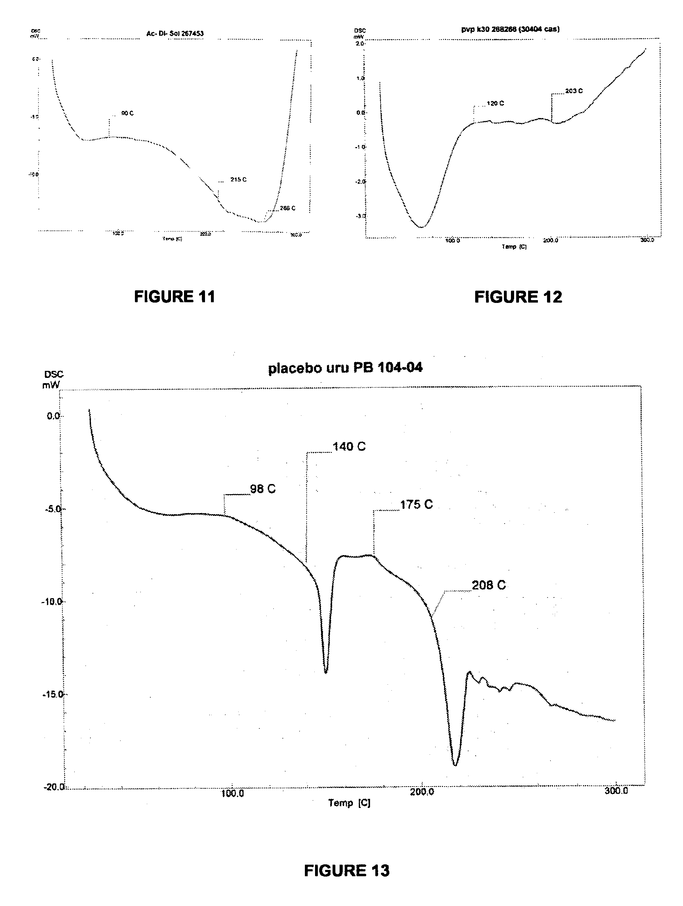

DSC - using a Shimadzu DSC-60 at 10 °C/min, with a flow of nitrogen at 30 ml/min as the purge gas - demonstrates the absence of the endothermic signal of fusion characteristic of the crystalline state of the drospirenone in the adsorbate obtained in accordance with Example 1.
The different inert components and drospirenone were analyzed by DSC before preparing the adsorbate of drospirenone and ethynylestradiol, in accordance with Example 1. Figure 5 shows a graph of the DSC of lactose monohydrate alone; Figure 6 shows a graph of the DSC of drospirenone in the crystalline state (temperature of the beginning of the endotherm To = 198,9 ± 0,8°C). Figure 7 shows a graph of the DSC of magnesium stearate; Figure 8 shows a graph of the DSC of corn starch; Figure 9 shows a graph of the DSC of pregelatinized starch; Figure 10 shows a graph of the DSC of yellow iron oxide; Figure 11 shows a graph of the DSC of sodic croscarmellose (AC-Di-Sol); Figure 12 shows a graph of the DSC of Povidone K30.
Figure 13 shows a graph of the DSC of the mixture of inert excipient components or placebo with the absence of drospirenone and ethynylestradiol.

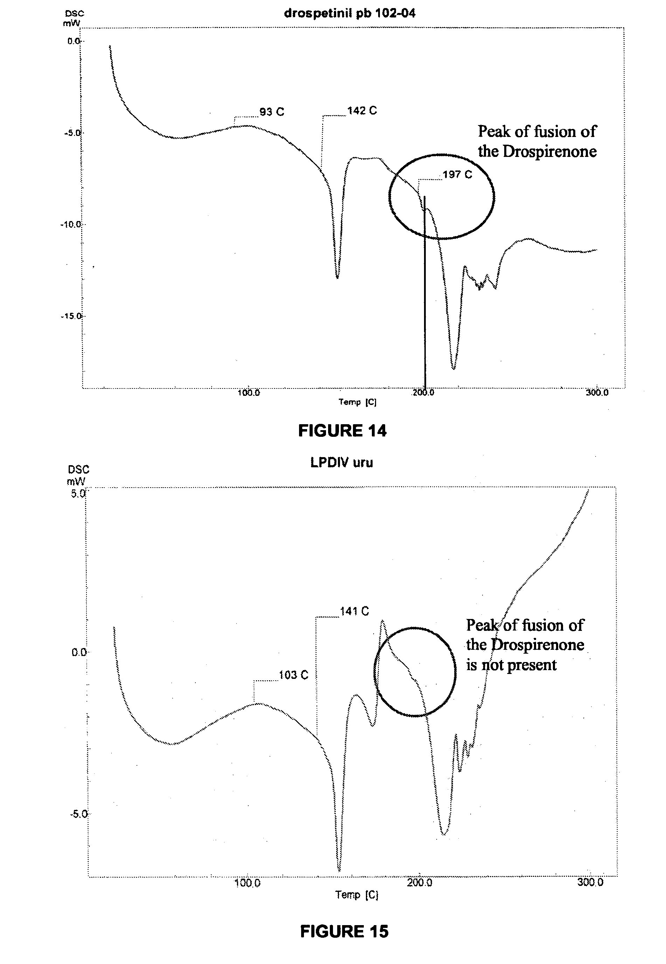

Figure 14 shows a graph of the DSC of the mixture of the components of the adsorbate in the state of simple mixture, where the signal of fusion of drospirenone in the crystalline state (endotherm of fusion at 197°C)has been particularly marked.
Figure 15 shows a graph of the DSC of the granulate obtained in Example 1 which contains drospirenone and ethynylestradiol adsorbed on inert excipients where one notes the lack of the peak of fusion of the drospirenone in the crystalline state. The lack of the peak indicates the loss of the crystalline state of drospirenone as a result of the process applied in Example 1, where by subjecting the granulate to drying, the presence of the polyvinylpyrrolidone in the drospirenone and ethynylestradiol solution acts through the formation of a tridimensional polymer structure, preventing the crystalline growth of both active compounds, so that the drugs are deposited on the excipient particles in an amorphous state.

### EXAMPLE 4

### Comparative dissolution profiles of drospirenone

Drospirenone dissolution profiles were obtained from compressed tablets obtained according to Example 1 and "methanol only" comparative example 2B using a dissolution apparatus designated in the U.S. Pharmacopoeia, Edition number 27, Apparatus 2, at 50 rpm and 900 ml of distilled water at 37°C. Similarly, the dissolution profiles of drospirenone were obtained from tablets containing drospirenone in a crystalline state with different sized particles. The results of the amount of drug dissolved at different times are summarized in the following table:

**Table 2**

| | % of drospireno ne dissolved | | | | | |
|---|---|---|---|---|---|---|
| Time (minutes) | 0 | 10 | 20 | 30 | 45 | 60 |
| LP01 | 0 | 31,2% | 48,0% | 59,9% | 69,9% | 77,8% |
| LP02 | 0 | 26.7% | 48,6% | 61,0% | 72,7% | 71,7% |
| LP03 | 0 | 32,6% | 56,2% | 70,7% | 74,3% | 80,5% |
| LPDIVuru | 0 | 80,5% | 97,0% | 101,0% | 102,0% | 102,0% |
| LPDISpray | 0 | 92% | 96% | 95% | 95% | 95% |
| LPDIMet-16ml | 0 | 22% | 29% | 33% | 38% | 42% |
| LPDIMet-154ml | 0 | 56% | 73% | 80% | 86% | 88% |

### Samples:

LP01: Batch of tablets with non-micronized, crystalline drospirenone with an average particle size of> 100 µm
LP02: Batch of tablets with non-micronized, crystalline drospirenone with an average particle size = 100 µm
LP03: Batch of tablets with non-micronized, crystalline drospirenone with an average particle size = 70 µm
LPDIVuru and LPDISpray: Batch of tablets with adsorbates of drospirenone and ethynylestradiol in amorphous form obtained according to Example 1 (i.e. use of mixture of methylene chloride and methanol). For LPDIVuru the granulation was done in a planetary-type mixer and kneaded and for LPDISpray the granulation was done in a fluid bed dryer and sprayed.
LPDIMet-16ml and LPDIMet-154ml: Batch of tablets with adsorbates of drospirenone and ethynylestradiol in amorphous form obtained according to comparative Example 2B (i.e. use methanol alone). The first is where 16 ml methanol was used (same amount as in example 1. Drospirenone was not completely dissolved. See example 2B) and the latter is where 154 ml methanol was used in order to completely dissolve drospirenone.

For a better interpretation of the results shown in Table 2, the results are also shown in Figure 17. From this figure, one can observe that the batches of tablets of drospirenone in crystalline state, with different sizes of particles, show a very slow dissolution rate. However the LPDIVuru batch, in accordance with the present invention, shows a dissolution rate for drospirenone of at least 80% within the 20 minutes.

Further, from Table 2 it can be seen that USE OF METHYLENE CHLORIDE AS SOLVENT GIVES SIGNIFICANT BETTER RESULTS COMPARED TO USE OF ONLY METHANOL AS SOLVENT.

### EXAMPLE 5

### Comparative dissolution profiles of ethynylestradiol

Ethynylestradiol dissolution profiles were obtained from compressed tablets obtained according to Example 1 and "methanol only" comparative example 2B using a dissolution apparatus designated in the U.S. Pharmacopoeia, Edition number 27, Apparatus 2, at 50 rpm and 900 ml of distilled water at 37°C. Similarly, the dissolution profiles of ethynylestradiol were obtained from tablets containing ethynylestradiol in a crystalline state with different sizes of particles. The results of the amount of drug dissolved at different times are summarized in the following table:

**Table 3**

| | % of ethynylest radiol dissolved | | | | | |
|---|---|---|---|---|---|---|
| Time (minutes) | 0 | 10 | 20 | 30 | 45 | 60 |
| LP01 | 0 | 10,0% | 14,5% | 21,0% | 32,0% | 45,0% |
| LP02 | 0 | 25,0% | 35,4% | 45,6% | 52,0% | 60,5% |
| LP03 | 0 | 33,1% | 42,0% | 55,5% | 63,0% | 72,5% |
| LPDIVuru | 0 | 75,9% | 86,0% | 92,0% | 100,1% | 101,8% |
| LPDISpray | 0 | 85% | 89,5% | 92,3% | 95,3% | 102,3% |
| LPDIMet-16ml | 0 | 70% | 75,3% | 81% | 95% | 99% |
| LPDIMet-154ml | 0 | 65% | 72,3% | 80,5% | 90,1% | 100,3% |

### Samples:

LP01: Batch of tablets with non-micronized, crystalline ethynylestradiol with an average particle size of> 100 µm
LP02: Batch of tablets with non-micronized, crystalline ethynylestradiol with an average particle size = 90 µm
LP03: Batch of tablets with non-micronized, crystalline ethynylestradiol with an average particle size = 80 µm
LPDIVuru and LPDISpray: Batch of tablets with adsorbates of drospirenone and ethynylestradiol in amorphous form obtained according to Example 1 (i.e. use of mixture of methylene chloride and methanol). For LPDIVuru the granulation was done in a planetary-type mixer and kneaded and for LPDISpray the granulation was done in a fluid bed dryer and sprayed.
LPDIMet-16ml and LPDIMet-154ml: Batch of tablets with adsorbates of drospirenone and ethynylestradiol in amorphous form obtained according to comparative Example 2B (i.e. use methanol alone). The first is where 16 ml methanol was used (same amount as in example 1 - drospirenone not completely dissolved) and the latter is where 154 ml methanol was used in order to completely dissolve drospirenone.

For a better interpretation of the results shown in Table 3, the results are also shown in Figure 18. From this figure, it can be observed that the batches of tablets of ethynylestradiol in crystalline state, with different sizes of particles, show a very slow dissolution rate. However, the LPDIVuru batch, in accordance with the present invention, shows a dissolution rate for ethynylestradiol of at least 80% within the 20 minutes.

Further, from Table 3 it can be seen that USE OF METHYLENE CHLORIDE AS SOLVENT GIVES BETTER RESULTS COMPARED TO USE OF ONLY METHANOL AS SOLVENT.

### EXAMPLE 6

Due to its low aqueous solubility, Drospirenone and Ethinylestradiol often shows low dissolution rate. The enhancement of rate of dissolution of poorly water-soluble drugs remains one of the most challenging aspects of drug product development. The solid dispersion of poorly water-soluble drugs in water-soluble surface-active carriers enhances drug dissolution and bioavailability.
The nature of Drospirenone and Ethinylestradiol dispersed in the matrix of polyvinylpyrrolidone K-30 was studied.

In this comparative example was example 1 repeated but made without use of Povidine (polyvinylpyrrolidone K-30) in the dissolution step. Microphotography by electronic microscopy was obtained. An Electronic Microscopic property of UNLP (Universidad Nacional de La Plata- Argentina) was used in order to obtain the microphotographs.

The microphotograph images of Drospirenone and Ethynylestradiol crystals obtained by the same manner as in Example I but without polyvinylpyrrolidone K-30 showed that there were micro-crystals precipitate on the excipients. On the contrary, for microphotograph of granules with Drospirenone and Ethinylestradiol obtained by wet granulation as in Example 1 (with polyvinylpyrrolidone K-30: matrix of solid dispersion), the image did not exhibit micro-crystals precipitate on the excipients.

Further, making the Dissolution Test with the USP's apparatus as aforementioned, the results indicated that the Drospirenone and Ethinylestradiol solid dispersion is released faster from the solid dispersion (with polyvinylpyrrolidone K-30) than from the pure crystalline drug (without polyvinylpyrrolidone K-30).

Accordingly, an improved product is obtained by adding a water soluble polymer such as polyvinylpyrrolidone K-30 (Povidone) in a manner as described herein.

## Claims

1. A pharmaceutical composition that comprises drospirenone and ethynylestradiol in amorphous form, adsorbed on a therapeutically inert solid support, in combination with pharmaceutically acceptable excipients, **characterized by** that the composition comprises measurable amounts of the volatile solvent methylene chloride.

2. The pharmaceutical composition in accordance with claim 1, **characterized by** that the composition comprises measurable amounts of both of the volatile solvents methylene chloride and methanol.

3. The pharmaceutical composition in accordance with claims 1 or 2, wherein the inert solid support is chosen from among particles of selected from the group consisting of corn starch, pregelatinized starch, lactose, sodic croscarmellose, yellow iron oxide and polyvinylpyrrolidone or mixtures of one or more of these, in combination with pharmaceutically acceptable excipients.

4. The pharmaceutical composition in accordance with any of the claims 1 to 3, **characterized in that** at least 80% of drospirenone and at least 80% of the ethynylestradiol dissolve from said composition within 20 minutes in a test carried out in a dissolution apparatus according to the U.S. Pharmacopoeia, Edition number 27, Apparatus 2, in 900 ml of distilled water at 37°C and stirred at 50 rpm.

5. A method for the preparation of a pharmaceutical composition, of any of the preceding claims, that comprises following steps:
i) dissolving drospirenone and ethynylestradiol in a volatile solvent or mixture of volatile solvents, wherein at least one of the volatile solvent is methylene chloride,
ii) optionally, adding a water-soluble polymer,
iii) mixing until dissolution,
iv) applying the resulting solution onto a base of solid particles that are therapeutically inert,
v) drying the obtained granulated adsorbate.

6. The method of claim 5, wherein the volatile solvent is a mixture of methylene chloride and methanol.

7. The method of claim 6, wherein the mixture of methylene chloride and methanol is a [9 to 4]:[3 to 0.5] v/v mixture of methylene chloride/methanol.

8. The method of claim 7, wherein the mixture of methylene chloride is a 6:1 v/v mixture of methylene chloride/methanol.

9. The method of any of claims 5 to 8, wherein there is step two of claim 5 is added a water-soluble polymer.

10. The method of claim 9, wherein the water-soluble polymer is polyvinylpyrrolidone.

11. The method of any of claims 5 to 10, wherein the solid support, that is therapeutically inert, is chosen from among particles of corn starch, pregelatinized starch, lactose, sodic croscarmellose, yellow iron oxide, polyvinylpyrrolidone or mixtures of one or more of the same.

12. The method of any of claims 5 to 11, wherein in addition, the dried granulated adsorbate is combined with pharmaceutically acceptable excipients for the compression of the tablets.

## Patentansprüche

1. Pharmazeutische Zusammensetzung, die auf einem therapeutisch inerten festen Trägermaterial adsorbiertes Drospirenon und Ethinylöstradiol in amorpher Form in Kombination mit pharmazeutisch verträglichen Hilfsstoffen enthält, **dadurch gekennzeichnet, dass** die Zusammensetzung messbare Mengen des flüchtigen Lösungsmittels Methylenchlorid enthält.

2. Pharmazeutische Zusammensetzung nach Anspruch 1, **dadurch gekennzeichnet, dass** die Zusammensetzung messbare Mengen der beiden flüchtigen Lösungsmittel Methylenchlorid und Methanol enthält.

3. Pharmazeutische Zusammensetzung nach Anspruch 1 oder 2, worin das inerte feste Trägermaterial aus Partikeln aus der Gruppe, die aus Speisestärke, Quellstärke, Laktose, Croscarmellose-Natrium, Eisen(III)-oxidhydroxid und Polyvinylpyrrolidon besteht, oder Mischungen eines oder mehrerer davon ausgewählt wird, kombiniert mit pharmazeutisch verträglichen Hilfsstoffen.

4. Pharmazeutische Zusammensetzung nach einem der Ansprüche 1 bis 3, **dadurch gekennzeichnet, dass** in einem Test, der in einem Lösungsprüfgerät nach der U.S. Pharmacopoeia, Ausgabe Nummer 27, Gerät 2, in 900 ml destilliertem Wasser bei 37°C und mit einer Umrührgeschwindigkeit von 50 Umdrehungen pro Minute ausgeführt wird, mindestens 80% des Drospirenons und mindestens 80% des Ethinylöstradiols aus der besagten Zusammensetzung innerhalb von 20 Minuten in Lösung übergehen.

5. Verfahren zur Herstellung einer pharmazeutischen Zusammensetzung, nach einem der vorstehenden Ansprüche, das folgende Verfahrenschritte umfasst:
i) Lösen des Drospirenons und Ethinylöstradiols in einem flüchtigen Lösungsmittel oder einer Mischung aus flüchtigen Lösungsmitteln, worin mindestens eines der flüchtigen Lösungsmittel Methylenchlorid ist,
ii) wahlweise Zugeben eines wasserlöslichen Polymers
iii) Mischen bis zur Auflösung,
iv) Auftragen der entstandenen Lösung auf ein Trägermaterial aus festen Partikeln, die therapeutisch inert sind,
v) Trocknen des erhaltenen gekörnten Adsorbats.

6. Verfahren nach Anspruch 5, bei dem das flüchtige Lösungsmittel eine Mischung aus Methylenchlorid und Methanol ist.

7. Verfahren nach Anspruch 6, bei dem die Mischung aus Methylenchlorid und Methanol eine [9 bis 14]:[3 bis 0,5] V/V Methylenchlorid/Methanol-Mischung ist.

8. Verfahren nach Anspruch 7, bei dem die Methylenchloridmischung eine 6:1 V/V Methylenchlorid/Methanol-Mischung ist.

9. Verfahren nach einem der Ansprüche 5 bis 8, bei dem in Verfahrensschritt zwei des Anspruchs 5 ein wasserlösliches Polymer zugegeben wird.

10. Verfahren nach Anspruch 9, bei dem das wasserlösliche Polymer Polyvinylpyrrolidon ist.

11. Verfahren nach einem der Ansprüche 5 bis 10, bei dem das feste Trägermaterial, das therapeutisch inert ist, aus Partikeln der Speisestärke, Quellstärke, Laktose, Croscarmellose-Natrium, Eisen(III)-oxidhydroxid, Polyvinylpyrrolidinon oder Mischungen aus einem oder mehreren davon ausgewählt wird.

12. Verfahren nach einem der Ansprüche 5 bis 11, bei dem zusätzlich das getrocknete gekörnte Adsorbat mit pharmazeutisch verträglichen Hilfsstoffen für die Tablettierung kombiniert wird.

## Revendications

1. Composition pharmaceutique qui comprend de la drospirénone et de l'éthynylestradiol sous forme amorphe, adsorbés sur un support solide inerte du point de vue thérapeutique, en combinaison avec des excipients acceptables sur le plan pharmaceutique, **caractérisée en ce que** la composition comprend des quantités mesurables du solvant volatil chlorure de méthylène.

2. Composition pharmaceutique selon la revendication 1, **caractérisée en ce que** la composition comprend des quantités mesurables de l'un et l'autre solvants volatils chlorure de méthylène et méthanol.

3. Composition pharmaceutique selon la revendication 1 ou 2, dans laquelle le support solide inerte est choisi parmi des particules sélectionnées dans le groupe constitué de l'amidon de maïs, l'amidon prégélatinisé, le lactose, la croscarmellose sodique, l'oxyde de fer jaune et la polyvinylpyrrolidone ou des mélanges d'un ou plusieurs de ceux-ci, en combinaison avec des excipients acceptables sur le plan pharmaceutique.

4. Composition pharmaceutique selon l'une quelconque des revendications 1 à 3, **caractérisée en ce qu'**au moins 80 % de la drospirénone et au moins 80 % de l'éthynylestradiol se dissolvent de ladite composition dans les 20 minutes dans un essai effectué dans un appareil de dissolution selon la Pharmacopée U.S., Numéro d'édition 27, Appareil 2, dans 900 mL d'eau distillée à 37 °C et sous agitation à 50 tr/min,

5. Procédé pour la préparation d'une composition pharmaceutique, selon l'une quelconque des revendications précédentes, lequel comprend les étapes suivantes :
i) une dissolution de drospirénone et d'éthynylestradiol dans un solvant volatil ou un mélange de solvants volatils, dans lequel au moins l'un des solvants volatils est du chlorure de méthylène,
ii) un ajout éventuel d'un polymère hydrosoluble,
iii) un mélange jusqu'à dissolution,
iv) une application de la solution résultante sur une base de particules solides qui sont inertes du point de vue thérapeutique,
v) un séchage de l'adsorbat granulé obtenu.

6. Procédé selon la revendication 5, dans lequel le solvant volatil est un mélange de chlorure de méthylène et de méthanol.

7. Procédé selon la revendication 6, dans lequel le mélange de chlorure de méthylène et de méthanol est un mélange [9 à 4]:[3 à 0,5] volume/volume de chlorure de méthylène/méthanol.

8. Procédé selon la revendication 7, dans lequel le mélange de chlorure de méthylène est un mélange 6:1 volume/volume de chlorure de méthylène/méthanol.

9. Procédé selon l'une quelconque des revendications 5 à 8, dans lequel on ajoute à l'étape deux de la revendication 5 un polymère hydrosoluble.

10. Procédé selon la revendication 9, dans lequel le polymère hydrosoluble est de la polyvinylpyrrolidone.

11. Procédé selon l'une quelconque des revendications 5 à 10, dans lequel le support solide, qui est inerte du point de vue thérapeutique, est choisi parmi des particules d'amidon de maïs, d'amidon prégélatinisé, de lactose, de croscarmellose sodique, d'oxyde de fer jaune, de polyvinylpyrrolidone ou des mélanges d'un ou plusieurs de ceux-ci.

12. Procédé selon l'une quelconque des revendications 5 à 11, dans lequel en outre, l'adsorbat granulé séché est combiné avec des excipients acceptables sur le plan pharmaceutique en vue de la compression des comprimés.
